# EUROPEAN PATENT SPECIFICATION

(11) **EP 2 111 822 B1**
(45) Date of publication and mention of the grant of the patent: **06.08.2014**
(21) Application number: 09158311.2
(22) Date of filing: 21.04.2009
(51) Int. Cl.: A61F 2/16

(54) **Intraocular lens with a toric optic**
Intraokularlinse mit einer torischen Optik
Lentille intraoculaire avec une optique torique

(30) Priority: 21.04.2008 NL 2001503
(43) Date of publication of application: 28.10.2009
(73) Proprietor: Oculentis Holding B.V., 6961 LZ Eerbeek (NL)
(72) Inventor: Wanders, Bernardus Franciscus Maria, 6986 AH, Angerlo (NL)
(74) Representative: Nederlandsch Octrooibureau

(56) References cited:
- WO-A-2005/055875
- WO-A-2006/136424
- US-A1- 2003 060 880
- US-A1- 2006 142 855
- US-A1- 2008 004 698

## Description

### Background of the invention

The invention relates to an intra-ocular lens (IOL).
A conventional intraocular lens is defined by rotational symmetric, spheric or aspheric curved surfaces, often on both sides of the lens. In that case, the optical axes lie centred in relation to one another. The incident light rays are consequently focused upon a point on the retina. Such a lens is centred in the capsular sac or the sulcus by peripheral aids, also known as haptics. The conventional lens with rotational symmetric curved surfaces is not suitable for correcting an astigmatic refractive error of the cornea.

By designing two of these surfaces, not rotationally symmetric, but toric or astigmatic, the incident light rays can be focused perfectly on the retina. In the toric intra-ocular (TIOL) lens according to the prior art, the thickness of the edge is kept constant. As a result, the optically effective surface becomes very elliptic. A drawback here is that, in the case of higher order astigmatic aberrations, the effective optical zone will become too small, as a result of which the patient will suffer from adverse side effects.

US2003/0060880, for example, describes a toric intraocular lens, wherein one side is provided with a toric surface with axis markers. Here, the lens is made from one piece. The resulting surface is elliptic. Various lens diameters refer to lenses on various sides of the IOL, i.e. on the anterior side and on the posterior side of the IOL.

WO2006136424A1 describes a bi-toric intraocular lens, wherein the toric surfaces are formed on both sides of the lens. These toric surfaces are formed aspheric. Because the toricity is preferably distributed equally between both surfaces, this particular embodiment retains a larger effective optical zone in the meridian with the steepest curvature. The disadvantages of this method are the higher costs of production and the accidental introduction of errors due to the fact that, during production, two identical lenses need to be aligned opposite one another and relative to one another. Moreover, in this case the resulting lens surface has an elliptic circumference. The flattest curvature defines the largest diameter of the lens. The diameter of the lens is therefore smaller along the axis of the largest curvature.

US-2008/0004698 describes the manufacture of the IOL in general terms. One side of the IOL may be provided with a toric lens. Here, account is taken in the design of the IOL of the aberration that may result from the surgical procedure that is performed to implant the IOL.

As indicated, in the case of the known embodiments in the prior art, the size of the effective optic, i.e. the surface and diameter, is defined by the degree of toricity of the lens and can therefore not be adjusted to a desired diameter beforehand. In the known TIOL, the smallest diameter of the optical zone is defined by the degree of toricity. An example is a lens of dioptre 20 and an optical zone of 5.5 mm on the flattest meridian and a toricity for the steepest meridian of dioptre 4. The smallest optical zone will then only amount to approximately 4.75 mm.

Therefore, there is room for the improvement of the intraocular lenses long known in the prior art.

### Summary of the invention

The object of the invention is to provide an alternative to the known intraocular lens.

Additionally or alternatively, the invention further aims to provide an improved intraocular lens.

Furthermore, an additional or alternative object of the invention is to provide an intraocular lens with a larger optical zone.

To this end, the invention provides an intraocular lens (IOL) with a toric optic for the correction of astigmatism, with on one side of a lens a most curved meridian with a most curved lens curvature and a flattest meridian with a lens curvature which is flatter than the most curved lens curvature, wherein an effective optical zone in the most curved meridian is greater than or practically equal to the effective optical zone in the flattest meridian.

In this manner, the effective optical diameters of a toric IOL can be made adjustable at both the steepest and flattest meridians, regardless of the toricity in the lens. It may even be possible to design the optical area almost circular. In the prior art, the effective lens surface appeared to be elliptic, as a result of which it has been demonstrated that the effective surface becomes considerably smaller. One apparently chose to allow the edge of the lens to connect to the haptic.

The other side of the IOL may, for example, be either toric, bifocal or multifocal, rotational symmetric, or have any other form of optical correction.

The intraocular lens comprises a transition zone between the toric optic and the further intraocular lens. Because of the presence of the transition zone, the diameter of the optical zone no longer needs to be dependent on the curvature of the optic.

In an embodiment, the lens circumference of the toric optic does not lie in a flat plane.

In an embodiment, the curve of the flattest meridian intersects a lens plane of the toric lens.

In an embodiment the diameter of the steepest meridian and of the flattest meridian are adjusted independently.

In an embodiment, the adjusted diameter of the flattest and steepest meridian is independent of the degree of toricity.

In an embodiment, contour variations at the point of the transition between the haptic and the toric optic run sinusoidally.

In an embodiment, contour variations at the point of the transition between the haptic and the toric optic are defined by a NURBS.

Due to these choices of curvature, the transition zone may run smoothly without adverse boundary effects occurring.

In an embodiment the toric optic is aspheric.

In an embodiment, the posterior side is provided with a substantially circumferential sharply defined edge.

In an embodiment the intraocular lens further comprises a bifocal optic, a multifocal optic or an aspheric optic.

In an embodiment the intraocular lens further comprises a marker on an optic for, in an embodiment the marker comprises a marker line at the point of the flattest meridian. This enables the IOL with toric optic to be correctly aligned in an eye.

In an embodiment the intraocular lens further comprises haptics, which, in an embodiment, are provided with an orientation marker.

In an embodiment the toric optic is formed by Fast tool means.

The invention further relates to an intraocular lens (IOL) with a toric optic for the correction of astigmatism, with on one side of a lens a most curved meridian with a most curved lens curvature and a flattest meridian with a flattest lens curvature, which is flatter than the most curved lens curvature, wherein the most curved meridian defines a maximum diameter lens.

Alternatively, the invention further relates to an intraocular lens (IOL) with a toric optic for the correction of astigmatism, with on one side of a lens a most curved meridian with a most curved lens curvature and a flattest meridian with a flattest lens curvature that is flatter than the most curved lens curvature, comprising a transition zone between the toric optic and the further intraocular lens.

Alternatively, the invention relates to an intraocular lens, comprising a toric optic and an almost entirely circumferential sharply defined outer edge on a posterior side of the intraocular lens.

Alternatively, the invention relates to a method for producing an intraocular lens, comprising the following steps:
- calculating a first curvature of a toric optic;
- calculating a second curvature of the toric optic;
- selecting a diameter of the toric optic at the point of the first curvature and of the second curvature;
- calculating the course of a transition zone to align the first and second curvatures of the optic to a common lens plane.

In an embodiment the method for the production of an intraocular lens further comprises the step of calculating the third curvature of an opposing optic disposed on the other side of the intraocular lens.

This gives rise to the advantages previously indicated.

A further aspect of the invention relates to the posterior side of an intraocular lens. An IOL is generally implanted in the capsular bag or the sulcus. During removal of the original crystalline lens, residual tissue is left behind which could give rise to posterior capsular opacification ("posterior capsular opacification", PCO). In the prior art, it is common practice to provide the posterior side of an IOL with an almost entirely circumferential sharply defined edge so as to guard against the occurrence of posterior capsular opacification ("posterior capsular opacification", PCO). Numerous embodiments thereof are known for rotational symmetric IOL, for example, such as those disclosed in US5171320, US5693093, US6162249 and EP1831746.

PCO also appears to occur in toric intraocular lenses. To this end, the invention provides an intraocular lens, comprising a toric optic and an almost entirely circumferential sharply defined outer edge on the posterior side of the intraocular lens.

It will be clear that the features of an IOL, as disclosed herein, may be applied in conjunction with an IOL as disclosed in PCT/NL2006/050152, PCT/NL2006/050142 or PCT/NL2006/050148 by the same applicant.

Furthermore, it will also be clear that a reading aid or any other known optically functional element or other functional aid may also be applied in conjunction with the IOL described in this present document.

The invention further relates to an intraocular lens provided with one or more of the characteristic measures described in the appended descriptions and/or in the appended drawings.

Furthermore, it will be clear that the various aspects and features disclosed herein may be applied conjunctively and that each feature may be considered as being individually eligible for a divisional patent application.

### Brief description of the figures

In the appended figures an embodiment of an intraocular lens is shown, wherein:
Fig. 1 shows a front view of an IOL according to the prior art;
Fig. 2 shows a side view of the IOL of fig. 1;
Fig. 3 shows the IOL of fig. 1 in perspective view;
Fig. 4 shows a perspective view of the front side of a toric IOL according to an embodiment of the invention;
Fig. 5 shows a perspective view of the rear side or the posterior side of the IOL of fig. 4 with an almost entirely circumferential sharply defined outer edge for the prevention of PCO;
Fig. 6 shows a perspective view of a partial cross-section of the IOL of fig. 4;
Fig. 7 shows a rear view, i.e. from the posterior side of the IOL of fig. 4;
Fig. 8 shows a front view of the anterior side of the IOL of fig. 4, wherein the flattest meridian is located at the point of the markers;

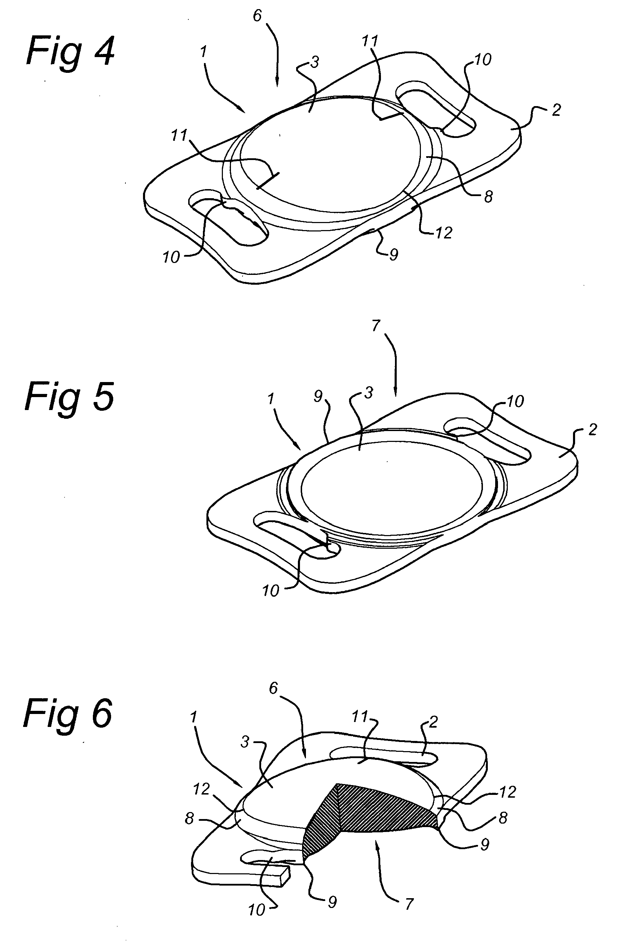

Fig. 6 shows a side view of the IOL of fig. 4;

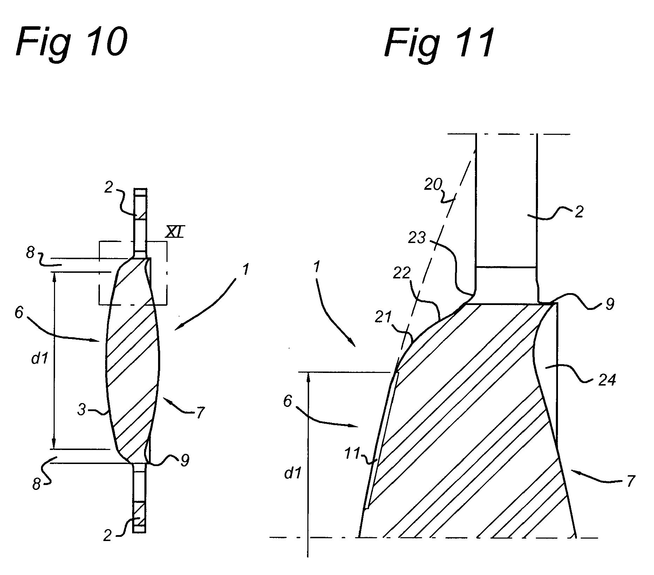

Fig. 10 shows a cross section of the IOL of fig. 4;
Fig. 11 shows a detail of fig. 10 as indicated;

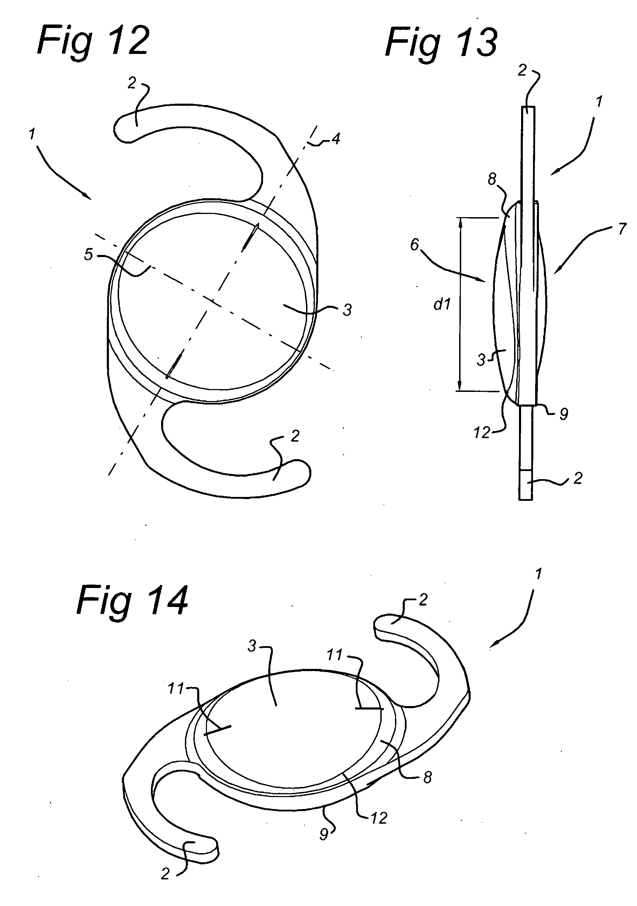

Fig. 12 shows an embodiment of the IOL according to the invention with alternative haptics;
Fig. 13 shows a side view of the IOL of fig. 12, and
Fig. 14 shows a perspective view of the IOL of fig. 12.

### Description of the embodiments

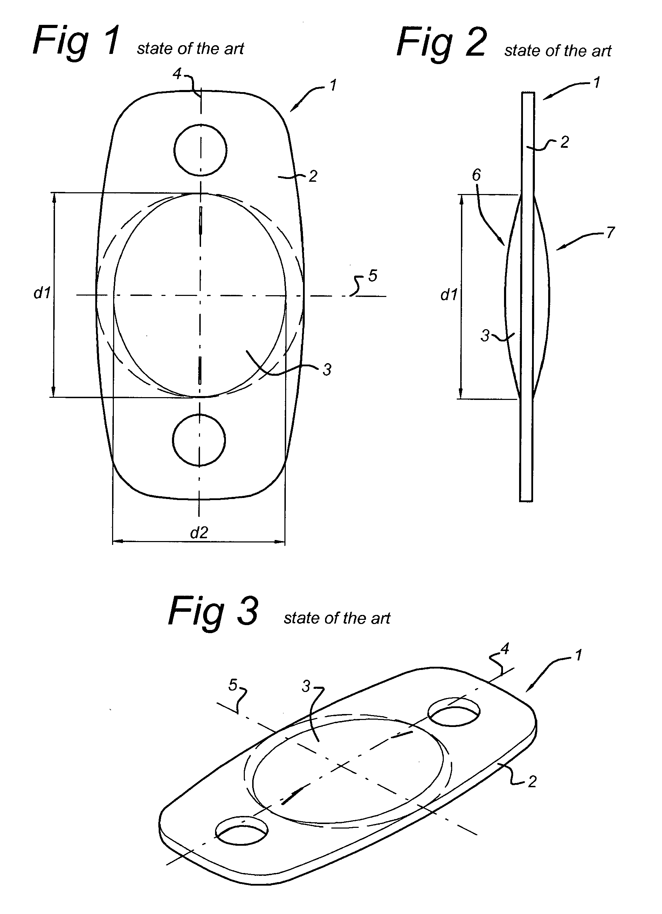

Figures 1 to 3 show an IOL according to the prior art. Here, the IOL 1 has a haptic 2 in the form of a plate-shaped haptic ("plate-haptic"). An optical element or optic 3 is attached to said plate-haptic. The optical element 3 has a flattest meridian, indicated by dotted line 4, and a most curved meridian indicated by dotted line 5. The curves of the flattest and/or steepest meridian can be formed aspheric to correct higher order aberrations, such as spheric aberration.

The optic or optical element 3 on the plate-shaped haptic 2 is designed so that the curve of the curvature intersects the upper surface of the plate-haptic 2, which is equal here to the lens surface of optic 3. The diameter of optic 3 and the diameter of the lens in the flattest meridian is accordingly d1, the smallest diameter at the steepest meridian therefore being d2. In the front view of figure 1, a circle is indicated by a dotted line which would arise if the diameter of the lens were to be equal along the entire outer perimeter of the optic 3. The full ellipse indicated now shows the circumference of the optic 3.

Figure 2 shows a side view of the IOL 1 according to figure 1. It can be seen that the curves of the curvature of the optic 3 intersect the upper surface of the plate-haptic 2. In figure 3 the IOL 1 of figure 1 is shown in perspective view. Here also, a dotted line indicates the lens diameter that would otherwise arise if a rotational symmetric lens were to be applied. It is clearly seen in the figures that the principles of design of the lens give rise to a so called optical zone, disposed within the full ellipse indicated in fig 1, which is elliptic. The diameter d1 is generally approximately 5.5 mm, and d2 will be smaller. The average diameter of the human iris is 4.5 mm, but there are also people with a larger pupil. It is therefore obvious that, in the average human being, when a substantial variation in dioptre is required, for example, as illustrated in the foregoing description, marginal phenomena may occur due to the edge of the pupil converging with the edge of the optical zone.

Figure 4 shows a perspective view, as viewed from the front, of an embodiment of a toric IOL 1 according to the invention. Once again, this IOL 1 is provided with a plate-shaped haptic 2 or so-called "plate-haptic". In this figure, the circumference of the optical zone is indicated by the line 12. Furthermore, a transition zone from the edge or perimeter 12 to the upper surface of the plate of the plate-shaped haptic 2 is indicated by the reference numeral 8.

On the front side 6 of the lens 3, markers 11 are shown. These markers 11 are often formed as linear grooves. It is important when the toric IOL 1 is implanted in the eye that the markers 11 are aligned in relation to a chosen directional axis in the cornea. During the surgical procedure during which the IOL 1 is implanted in the eye, this directional axis of the cornea is measured and can be referenced, for example, by means of a temporary marker on the limbus of the eye. During the implantation of the IOL 1 the markers 11 of the IOL are then aligned in respect of, for example, the temporary markers formed on the limbus.

Figure 5 shows in perspective view, the rear side or posterior side 7 of an IOL 1 according to an embodiment of the invention. This view clearly shows a sharply defined circumferential outer edge 9. This circumferential sharply defined edge 9 is applied in order to prevent the occurrence of posterior capsular opacification (PCO). A possible optic 3 on posterior side 7 of the IOL can be formed spherically or aspheric, or, if so desired, be provided with a bifocal toric or multifocal optic. This also applies to the optic 3 on the front side 6 of the IOL 1.

Figure 6 shows a front view in perspective of the TIOL 1, as shown previously, wherein a portion is removed in order to make the cross section visible. In this cross-section it can be clearly seen that the circumferential sharply defined edge 9 in this embodiment encompasses the optical zone of the lens.

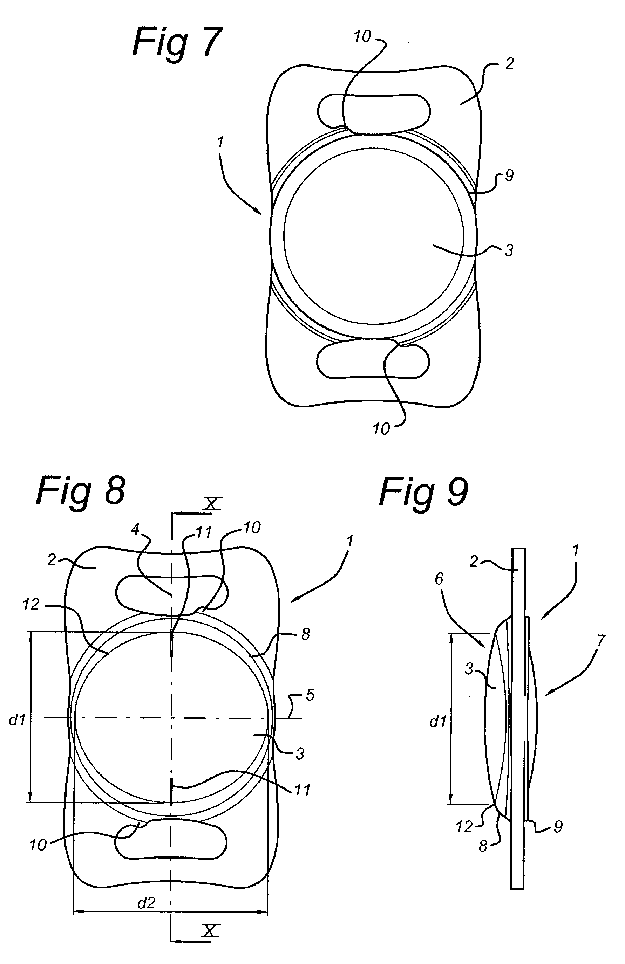

Figure 7 shows the rear side of the TIOL 1 or the posterior side 7 of the TIOL 1. In this case, it can be clearly seen that the sharp edge 9 here is an almost entirely circumferential sharply defined edge 9. In addition, orientation markers 10 are also shown. These orientation markers 10 can be used by a surgeon, after implantation, to determine whether the IOL is implanted in the eye with the correct side facing forwards.

Figure 8 shows a front view of the TIOL 1. In this view, the meridian 4 with the flattest curvature and the meridian 5 with the steepest curvature are shown. In this embodiment, the diameter d1 of the optic 3 in the flattest meridian 4 and the diameter d2 of the optic 3 in the most curved meridian 5 are almost identical. Consequently, the optical zone or optic 3 is essentially circular. The steepest meridian 5 of the TIOL can be maximally adjusted to the size of the total IOL. This size usually lies between 5.0 and 6.0 mm, with the preferred size being approximately 5.5 mm. If so desired, the diameter of the flattest meridian may be adjusted so that the size of the effective optical zone can be matched to the optimum size, regardless of the toricity.

The boundary or perimeter 12 of the lens is also indicated here with the full, essentially circular line 12. A transition zone 8 extending from this circumference is indicated, wherein the surface of the optic 3 transforms towards the surface of the haptic 2 which forms here the plane of the lens.

Figure 9 shows a side view of figure 8, wherein the diameter d1 is also indicated for the sake of clarity. The circle 12 and the transition zone 8 are also indicated. The transition zone will be described in more detail in figure 11.

Figure 10 shows a cross section of the TIOL 1 of figure 8 as indicated. The diameter d1 is also indicated here, as well as the transition zone 8.

Figure 11 shows an enlargement of the cross section of figure 10 as indicated. In figure 11 a continuation of the curvature of the anterior or anterior surface of the TIOL is indicated by dotted lines 20. Here, it can be clearly seen that the transition zone 8 runs sinusoidally from the lens 3 (in a zone 21), then more steeply in zone 22 and once again has a sinusoidal adjustment zone 23. The transition zone preferably aligns tangentially with the existing contour in order to form an absolutely smooth transition without any troublesome transition arising. In addition to a sinusoidal transition, this transition zone may also be defined by a spline or NURBS (Non Uniform Rational Bspline). Due to the presence of this special transition zone 8, the diameter of the flat zone is not determined by an alignment of the curvature with the surface, but by a transition zone.

Furthermore, this figure clearly shows an exemplary embodiment of the sharply defined edge 9 for the prevention of PCO. A further visional acuity of this outer edge occurs as a result of the deepening 24 at the edge of the lens. The curvature of the lens is chosen in such a manner that this is not located at the surface of the plate-shaped haptic 2, but further beneath the surface, ensuring that the cavity is formed, so that the sharply defined edge 9 provides visional acuity. The choice is such that the lens curvature ends at the position of optic 3 on the front side 6, and then continues to the sharply defined edge 9. This results in an additional visional acuity of edge 9.

Figure 12 shows an embodiment of the TIOL 1 according to the invention, provided here with a heterologous haptic 2. The further design of the optical zone, the alignment and the sharply defined edge on the posterior side correspond almost entirely to those of the figures 4 to 11.

The toric lens can be produced, for example, by means of a moulding process, wherein the lens is cast as a single piece from a mould suitable for that purpose. Alternatively, the lens can be formed by means of machining, from a suitable material. The materials may have hydrophilic or hydrophobic properties.

The toric surface of optic 3, together with the (in this case sinusoidal) transition zone 8 of haptic 2 to optic 3 and the markers 11, are produced, for example, by means of ultra-precise machining with a Fast Tool Servo (FTS). Such FTS means, for example, are known from WO2005043266 and are produced commercially by Precitec Inc. in the USA, Kinetic Ceramics Inc. and Contamac BV in the Netherlands.

It will be obvious that the foregoing description is given in order to illustrate how the preferred embodiments of the invention function and not to limit the range and scope of the invention. With reference to the foregoing disclosure, those skilled in the art will become readily aware of numerous alternative embodiments, all of which fall within the scope of the present invention.

## Claims

1. Intraocular lens (1) with an optic (3) having a toric lens side for the correction of astigmatism, said toric lens side having a most curved meridian (5) with a most curved lens curvature and a flattest meridian (4) with a lens curvature which is flatter than the most curved lens curvature, **characterized in that** said intraocular lens (1) comprises a transition zone (8) between an optical zone of said toric lens side and the further intraocular lens such that an effective optical zone in the most curved meridian (5) is larger or essentially equal to the effective optical zone in the flattest meridian (4).

2. Intraocular lens according to claim 1, wherein the circumference (12) of the optical zone of the toric lens side of the optic does not lie in a flat plane.

3. Intraocular lens according to any one of the preceding claims, wherein the diameter of the most curved meridian (5) and of the flattest meridian (4) are adjusted independently.

4. Intraocular lens according to claim 3, wherein the adjusted diameter of the flattest meridian (4) and most curved meridian (5) is independent of the degree of toricity.

5. Intraocular lens according to any one of the preceding claims, wherein contour variations in the transition zone (8) between the further intraocular lens and the toric lens side of the optic (3) run sinusoidally.

6. Intraocular lens according to any one of the preceding claims, wherein contour variations in the transition zone (8) between the further intraocular lens and the toric lens side of the optic (3) are defined by a non uniform rational Bspline.

7. Intraocular lens according to any one of the preceding claims, wherein a front side (6) of the optic is provided with said toric lens side and a posterior side (7) of the optic is provided with a substantially circumferential sharply defined outer edge (9).

8. Intraocular lens according to any one of the preceding claims, further comprising a marker (10; 11) for alignment of the toric lens side of the optic (3) in an eye,

9. Intraocular lens according to claim 8, wherein said marker (10; 11) comprises marker lines (11) at the point of the flattest meridian (4),

10. Intraocular leans according to claim 8 or 9, wherein said IOL further comprises haptics (2) which are provided with an orientation marker (10).

11. Intraocular lens according to any one of the preceding claims, wherein the optic (3) is formed by Fast tool means, by means of a machining operation.

12. Intraocular lens according to any one of the preceding claims, wherein the most curved meridian (5) defines a maximum diameter lens.

13. Method for the production of an intraocular lens according to any one of the preceding claims, said intraocular lens comprising an optic, comprising the steps of:
- calculating a first curvature of a toric lens side of said optic;
- calculating a second curvature of the toric lens side of the optic;
- selecting a diameter of the toric lens side of the optic at the point of the first curvature and of the second curvature;
- calculating the course of a transition zone to align the first and second curvatures of the optic to a common lens plane.

14. Method for the production of an intraocular lens according to claim 13, further comprising the step of calculating the third curvature of an opposing optic lens side on the other side of the intraocular lens.

## Patentansprüche

1. Intraokularlinse (1) mit einer Optik (3), welche eine torische Linsenseite zur Korrektur von Astigmatismus aufweist, wobei die torische Linsenseite einen am stärksten gekrümmten Meridian (5) mit einer am stärksten gekrümmten Linsenkrümmung sowie einen flachsten Meridian (4) mit einer Linsenkrümmung aufweist, die flacher als die am stärksten gekrümmte Linsenkrümmung ist,
**dadurch gekennzeichnet, dass** die Intraokularlinse (1) eine Übergangszone (8) zwischen einer optischen Zone der torischen Linsenseite aufweist, derart, dass eine effektive optische Zone in dem am stärksten gekrümmten Meridian (5) größer oder im Wesentlichen gleich der effektiven optischen Zone in dem flachsten Meridian (4) ist.

2. Intraokularlinse nach Anspruch 1, wobei der Umfang (12) der optischen Zone der torischen Linsenseite der Optik nicht in einer flachen Ebene liegt.

3. Intraokularlinse nach einem der vorhergehenden Ansprüche, wobei der Durchmesser des am stärksten gekrümmten Meridians (5) und des flachsten Meridians (4) unabhängig eingestellt sind.

4. Intraokularlinse nach Anspruch 3, wobei der eingestellte Durchmesser des flachsten Meridians (4) und des am stärksten gekrümmten Meridians (5) unabhängig vom Grad der Torizität ist.

5. Intraokularlinse nach einem der vorhergehenden Ansprüche, wobei Konturvariationen in der Übergangszone (8) zwischen der weiteren Intraokularlinse und der torischen Linsenseite der Optik (3) sinusförmig verlaufen.

6. Intraokularlinse nach einem der vorhergehenden Ansprüche, wobei Konturvariationen in der Übergangszone (8) zwischen der weiteren Intraokularlinse und der torischen Linsenseite der Optik (3) durch einen ungleichmäßigen rationalen B-Spline definiert sind.

7. Intraokularlinse nach einem der vorhergehenden Ansprüche, wobei eine Vorderseite (6) der Optik mit der torischen Linsenseite versehen ist und eine Rückseite (7) der Optik mit einem im Wesentlichen umlaufenden, scharf definierten Außenrand (9) versehen ist.

8. Intraokularlinse nach einem der vorhergehenden Ansprüche, ferner umfassend eine Markierung (10; 11) zur Ausrichtung der torischen Linsenseite der Optik (3) in einem Auge.

9. Intraokularlinse nach Anspruch 8, wobei die Markierung (10; 11) Markierungslinien (11) an der Stelle des flachsten Meridians (4) beinhaltet.

10. Intraokularlinse nach Anspruch 8 oder 9, wobei die IOL ferner eine Haptik (2) aufweist, welche mit einer Orientierungsmarkierung (10) ausgeführt ist.

11. Intraokularlinse nach einem der vorhergehenden Ansprüche, wobei die Optik (3) durch schnelle Werkzeugmittel mittels eines spanenden Vorgangs gebildet ist.

12. Intraokularlinse nach einem der vorhergehenden Ansprüche, wobei der am stärksten gekrümmte Meridian (5) eine Linse maximalen Durchmessers definiert.

13. Verfahren zur Herstellung einer Intraokularlinse nach einem der vorhergehenden Ansprüche, wobei die Intraokularlinse eine Optik umfasst, umfassend die Schritte:
- Berechnen einer ersten Krümmung einer torischen Linsenseite der Optik;
- Berechnen einer zweiten Krümmung der torischen Linsenseite der Optik;
- Wählen eines Durchmessers der torischen Linsenseite der Optik an der Stelle der ersten Krümmung und der zweiten Krümmung;
- Berechnen des Verlaufs einer Übergangszone, um die erste und die zweite Krümmung der Optik gegenüber einer gemeinsamen Linsenebene auszurichten.

14. Verfahren zur Herstellung einer Intraokularlinse nach Anspruch 13, ferner umfassend den Schritt des Berechnens der dritten Krümmung einer gegenüberliegenden optischen Linsenseite auf der anderen Seite der Intraokularlinse.

## Revendications

1. Lentille intraoculaire (1) avec une optique (3) ayant un côté de lentille torique pour la correction de l'astigmatisme, ledit côté de lentille torique ayant un méridien le plus incurvé (5) avec une courbure de lentille la plus incurvée et un méridien le plus plat (4) avec une courbure de lentille qui est plus plate que la courbure de lentille la plus incurvée, caracté-risée en ce que ladite lentille intraoculaire (1) comprend une zone de transition (8) entre une zone optique dudit côté de lentille torique et le reste de la lentille intraoculaire de sorte qu'une zone optique efficace dans le méridien le plus incurvé (5) soit plus grande ou essentielle-ment égale à la zone optique efficace dans le méridien le plus plat (4).

2. Lentille intraoculaire selon la revendica-tion 1, dans laquelle la circonférence (12) de la zone optique du côté de lentille torique de l'optique ne se trouve pas dans un plan plat.

3. Lentille intraoculaire selon l'une quelconque des revendications précédentes, dans laquelle les diamètres du méridien le plus incurvé (5) et du méridien le plus plat (4) sont ajustés de manière indépendante.

4. Lentille intraoculaire selon la revendica-tion 3, dans laquelle le diamètre ajusté du méridien le plus plat (4) et méridien le plus incurvé (5) est indépendant du degré de toricité.

5. Lentille intraoculaire selon l'une quel-conque des revendications précédentes, dans laquelle les variations de contour dans la zone de transition (8) entre le reste de la lentille intraoculaire et le côté de lentille torique de l'optique (3) s'étendent de manière sinusoïdale.

6. Lentille intraoculaire selon l'une quel-conque des revendications précédentes, dans laquelle les variations de contour dans la zone de transition (8) entre le reste de la lentille intraoculaire et le côté de lentille torique de l'optique (3) sont définies par une B-spline rationnelle non uniforme.

7. Lentille intraoculaire selon l'une quel-conque des revendications précédentes, dans laquelle un côté avant (6) de l'optique est pourvu dudit côté de lentille torique et un côté postérieur (7) de l'optique est pourvu d'un bord extérieur (9) nettement défini sensiblement circonférentiel.

8. Lentille intraoculaire selon l'une quel-conque des revendications précédentes, comprenant en outre un marqueur (10, 11) pour un alignement du côté de lentille torique de l'optique (3) dans un oeil.

9. Lentille intraoculaire selon la revendic-ation 8, dans laquelle ledit marqueur (10, 11) comprend des lignes de marqueur (11) au point du méridien le plus plat (4).

10. Lentille intraoculaire selon la revendica-tion 8 ou 9, dans laquelle ladite lentille intraoculaire comprend en outre des haptiques (2) qui sont pourvues d'un marqueur d'orientation (10).

11. Lentille intraoculaire selon l'une quel-conque des revendications précédentes, dans laquelle l'optique (3) est formée par des moyens formant outil rapide, au moyen d'une opération d'usinage.

12. Lentille intraoculaire selon l'une quel-conque des revendications précédentes, dans laquelle le méridien le plus incurvé (5) définit une lentille à diamètre maximum.

13. Procédé pour la production d'une lentille intraoculaire selon l'une quelconque des revendications précédentes, ladite lentille intra-oculaire comprenant une optique, comprenant les étapes :
- de calcul d'une première courbure d'un côté de lentille torique de ladite optique ;
- de calcul d'une deuxième courbure du côté de lentille torique de l'optique ;
- de sélection d'un diamètre du côté de lentille torique de l'optique au point de la première courbure et de la deuxième courbure ;
- de calcul de la trajectoire d'une zone de transition pour aligner les première et deuxième courbures de l'optique par rapport à un plan de lentille commun.

14. Procédé pour la production d'une lentille intraoculaire selon la revendication 13, comprenant en outre l'étape de calcul de la troisième courbure d'un côté de lentille optique opposé de l'autre côté de la lentille intraoculaire.
